# EUROPEAN PATENT SPECIFICATION

(11) **EP 2 825 660 B1**
(45) Date of publication and mention of the grant of the patent: **15.08.2018**
(21) Application number: 13761003.6
(22) Date of filing: 14.03.2013
(51) Int. Cl.: C01B 32/60, C12P 3/00, B01D 53/62, C01D 7/26, C01D 7/10

(54) **ENZYME ENHANCED PRODUCTION OF BICARBONATE COMPOUNDS USING CARBON DIOXIDE**
ENZYMVERSTÄRKTE HERSTELLUNG VON BICARBONATVERBINDUNGEN MIT KOHLENDIOXID
UTILISATION DE DIOXYDE DE CARBONE POUR AMÉLIORER LA PRODUCTION DE COMPOSÉS À BASE DE BICARBONATES PAR VOIE ENZYMATIQUE

(30) Priority: 14.03.2012 US 201261610838 P
(43) Date of publication of application: 21.01.2015
(73) Proprietor: CO2 Solutions Inc., Québec QC G2C 1T9 (CA)
(72) Inventor: VERSTEEG, Geert Frederik, NL-7522 PD Enschede (NL)
(74) Representative: Santarelli
(86) International application number: PCT/CA2013/050199
(87) International publication number: WO 2013/134879

(56) References cited:
- WO-A1-2008/072979
- WO-A1-2008/099252
- US-B1- 6 524 843
- US-B2- 7 579 185
- US-B2- 7 579 185
- US-B2- 7 727 374
- COLLETT JR ET AL.: 'Dissolved Carbonic Anhydrase for Enhancing Post-Combustion Carbon Dioxide Hydration in Aqueous Ammonia' ENERGY PROCEDIA vol. 4, 2011, pages 240 - 244, XP028212907
- CARGILL B ET AL.: 'Using Carbonic Anhydrase and Silica Nanomaterials in Carbon Dioxide Mitigation' FINAL PAPER, 2010 GOVERNOR'S SCHOOL OF ENGINEERING AND TECHNOLOGY RESEARCH JOURNAL, RUTGERS SCHOOL OF ENGINEERING 23 July 2010, XP055168026 Retrieved from the Internet: <URL:http:l/soe.rutgers.edu/fileslNano.pdf> [retrieved on 2013-05-15]
- PIERRE AC: '''Enzymatic Carbon Dioxide Capture'', International Scholarly Research Network, (ISRN)' CHEMICAL ENGINEERING vol. 2012, 2012, pages 1 - 22, XP055167483

## Description

### FIELD OF THE INVENTION

The present invention relates generally to a process for the production of bicarbonate compounds using CO₂ and more particularly to a process using enzyme as a biocatalyst to enhance the production of such bicarbonate compounds.

### BACKGROUND OF THE INVENTION

Sodium bicarbonate (or sodium hydrogen carbonate, NaHCO₃) is one of the most widely used bicarbonate compounds. Sodium bicarbonate has a long history of use in foodstuff, feed and industrial processes. Referred to as "baking soda", it is primarily used in cooking (baking), as a leavening agent. Thanks to its acidic and basic neutralizing properties, it is also used, for example, for raising the pH balance of water in swimming pools, spas or garden ponds. It has weak disinfectant properties, and it may be an effective fungicide against some organisms. Since sodium bicarbonate acts as a neutralizing agent, it can be used to absorb odors that are caused by strong acids.

In the medical field, sodium bicarbonate is useful, for example, as an antacid to treat acid indigestion and heartburn. Intravenous sodium bicarbonate solution is sometimes used for cases of acidosis, or when there are insufficient sodium or bicarbonate ions in the blood. It is used as well for treatment of hyperkalemia. Sodium bicarbonate has also been used in the treatment of tricyclic antidepressant overdose. It can also be applied topically as a paste to relieve insect bites.

In the field of personal hygiene, sodium bicarbonate is also used as an ingredient in some mouthwashes. It works as a mechanical cleanser on the teeth and gums, neutralizes the production of acid in the mouth and also acts as an antiseptic to help prevent infections. Sodium bicarbonate in combination with other ingredients can be used to make a dry or wet deodorant. It may also be used as a shampoo.

In the field of cleaning, sodium bicarbonate can be very effective when used as a cleaning and scrubbing agent alone or when added to detergents. It is commonly added to washing machines as a replacement for softener and also to remove odors from clothes. Sodium bicarbonate is also effective in removing heavy tea and coffee stains from cups when diluted with warm water.

As a biopesticide, sodium bicarbonate can be an effective for controlling fungus growth. It is registered as a biopesticide by the US Environmental Protection Agency.

The need for sodium bicarbonate is significant and there is a large worldwide market for its production.

Sodium bicarbonate can be produced in the middle stages of the soda ash (Na₂CO₃) production process often called the "Solvay Process". This process implies reacting sodium chloride, ammonia and carbon dioxide in water. In this process, calcium carbonate is used as the CO₂ source.

The first step of the "Solvay Process" is therefore the following reaction:

NaCI + NH₃+ CO₂ + H₂O → NaHCO₃ + NH₄Cl.

In industrial practice, the reaction is carried out by passing concentrated brine (NaCI) through two absorption towers. In the first, the strong brine is saturated with NH₃; while in the second, CO₂ bubbles up through the ammoniated brine, forming sodium bicarbonate (NaHCO₃) and ammonium chloride (NH₄Cl). Given that NaHCO₃ is less soluble than both NaCI and NH₄Cl in the ammonia (NH₃) buffered basic solution, the formed NaHCO₃ precipitates and is later filtered.

The CO₂ required for the process is produced by heating limestone at 950-1100°C through the following reaction:

CaCO₃ → CaO + CO₂.

The quicklime (CaO) formed from the limestone heating process is used to regenerate ammonia (NH₃) from NH₄Cl by-product of the Solvay process, according to the following equation:

2 NH₄Cl + CaO → 2 NH₃ + CaCl₂ + H₂O.

The regenerated ammonia is recycled back to the first brine absorption tower.

The sodium bicarbonate that precipitates out of the ammonium chloride solution is then filtered. Sodium carbonate (Na₂CO₃), which is the end product of the "Solvay Process" is then obtained by heating the sodium bicarbonate in a calciner at 160-230 °C as follows:

2 NaHCO₃ → Na₂CO₃ + H₂O + CO₂

The released CO₂ is also recycled back into the process.

Sodium bicarbonate may also be produced directly from sodium carbonate. In this process, sodium carbonate is dissolved into water to form a homogenous solution. The solution is fed to a bubble column where it is contacted with a CO₂ gas. The CO₂ gas is bubbled through the solution flowing through the column. Because of the high pH in the solution, the absorbed CO₂ reacts with the solution to produce bicarbonate ions according to the following reaction:

CO₂ + OH⁻ → HCO₃⁻.

The bicarbonate concentration reaches a level such that the solubility of the product is over the solubility value, and sodium bicarbonate precipitates. Overall, the reaction can be depicted as follows:

Na₂CO₃ + CO₂ + H₂O → 2 NaHCO₃ .

However, one of the problems in this process, when applied on an industrial scale, is that its efficiency towards converting the bubbled CO₂ is less than 100% because some of the CO₂ does not dissolve or absorb and thus is vented out of the bubble column. This may be called CO₂ "slip". The efficiency of CO₂ conversion often does not exceed 50 %.

Potassium bicarbonate (KHCO₃) is also a widely used bicarbonate product which has many different applications. It may be used as a fire extinguisher ingredient, and is more particularly efficient for Class B (flammable liquids and gases) and Class C (electrical) type fires. In the field of agriculture, it is used as a fertilizer. Potassium carbonate is also useful in the field of pharmaceuticals and foods. For example, it used as antacid, electrolyte replenisher and potassium supplement. KHCO₃ is often present as an excipient in pharmaceutical compositions. As with sodium bicarbonate, KHCO₃ is used in foods as a leavening agent. It may also serve as a color preservative in foods. Other examples of possible uses of KHCO₃ include as a household odour remover, an accelerator in fast setting cements, an additive in aqueous resin-based coatings and adhesives, a high-temperature polymer blowing agent, a detergent builder, a de-icer, and in hair and skin products.

Ammonium bicarbonate (NH₄HCO₃) is another bicarbonate product which is used in the food industry as a leavening agent for baked goods. It may be used as a nitrogen fertilizer, in the production of fire-extinguishing compounds, pharmaceuticals, dyes, or pigments. Ammonium bicarbonate is widely used in the plastic and rubber industry, in the manufacture of ceramics, in chrome leather tanning, and for the synthesis of catalysts. It is also used for buffering solutions of slightly alkaline pH during chemical purification, such as HPLC.

Cesium bicarbonate (CsHCO₃) is another bicarbonate product of interest. It is mainly used in chemical synthesis. For example, it is used as a base in organic chemistry. It is also used to make other cesium compounds.

Different ways for CO₂ capture have been developed over the past decades. One method uses a biocatalyst for enhancing CO₂ absorption. More particularly, US 6,524,843, discloses a process using a packed tower-type bioreactor containing the enzyme carbonic anhydrase. In the bioreactor, the carbon dioxide of the gaseous phase diffuses into the liquid phase and the immobilized carbonic anhydrase catalyses the hydration of the carbon dioxide, which forms hydrogen and bicarbonate ions according to the following equation:

CO₂ + H₂O ⇄ HCO₃⁻ + H⁺.

Technology for CO₂ capture from a CO₂ containing gas using carbonic anhydrase has also included precipitation of the ions by addition of a metal cation. The publication "Using carbonic anhydrase and silica nanomaterials in carbon dioxide mitigation" B. Cargill et al., published on 23.07.2010, discloses the conversion of carbon dioxide to bicarbonate which can then be converted to sodium bicarbonate for example. Carbon dioxide is reacted with water in the presence of carbonic anhydrase which is immobilized on silica nanoparticles. The resulting product, bicarbonate, can be reacted with sodium to form NaHCO₃. WO 2008/072979 relates to a process for capturing CO₂ from a gas effluent, wherein CO₂ is further desorbed from bicarbonate solids. The produced bicarbonate is reused to be converted back to carbonate. The objective is thus to minimize energy consumption for a regeneration process. A CO₂-containing gas is converted with an aqueous absorbent slurry comprising an inorganic alkali carbonate, bicarbonate and a promoter/catalyst. The precipitated bicarbonate is filtered, dewatered and then heated in a stripper for decomposition into carbonate and CO₂ release.

WO 2008/099252 relates to a process for converting CO₂ into methane. More particularly, the process involves the capture of CO₂ from a gas effluent, the desorption of CO₂ from a bicarbonate to produce CO₂ to be stored and eventually be converted into methane by an anaerobic digestion process. An aqueous solution of carbonate is contacted with the CO₂ containing gas in a first reactor (1A) where the carbonic anhydrase is immobilized. The bicarbonate-containing stream exiting the first reactor is sent to a precipitation reactor (1C) where solid carbonate is further added to obtain an oversaturated bicarbonate solution. The solution containing suspended solid bicarbonate is sent to a solid/liquid separation unit, and the thus separated bicarbonate is sent to a regeneration unit, where it is calcined to release CO₂. The carbonate produced in the regeneration unit is recovered and reused in the precipitation step.

The publication *"*Enzymatic Carbon Dioxide Capture", AC Pierre, International Scholarly Research Network (ISRN), Chemical Engineering, vol. 2012, pp 1-22) describes usual carbon dioxide captures using carbonic anhydrase enzymes.

There is a need for a technology that overcomes some of the problems and challenges of known techniques for producing bicarbonate compounds. For example, there is a need for a process for producing bicarbonate compounds, such as sodium bicarbonate, wherein the CO₂ conversion efficiency is increased.

### SUMMARY OF THE INVENTION

The present invention responds to the above mentioned need by providing a process for the production of bicarbonate compounds using CO₂ which is enhanced in the presence of an enzyme.

In one aspect, the present invention provides an enzyme enhanced process for the production of a bicarbonate compound from the corresponding carbonate and CO₂ according to claim 1.

More particularly, the present invention provides an enzymatic process for production of a bicarbonate compound, including:
contacting an aqueous solution including a dissolved carbonate and a carbonic anhydrase or an analogue thereof with a CO₂-containing gas to produce an enzyme and bicarbonate loaded stream;
separating the carbonic anhydrase or analogue thereof from the bicarbonate loaded stream;
converting the bicarbonate loaded stream into precipitated bicarbonate and a bicarbonate depleted stream, the precipitated bicarbonate including the bicarbonate compound;
wherein the precipitated bicarbonate is removed from the bicarbonate depleted stream in a thick slurry stream; and
wherein the process further comprises drying the thickened slurry stream to produce the bicarbonate compound.

In one optional aspect, the process produces a stable bicarbonate compound.

In another optional aspect, the carbonate is sodium carbonate and the bicarbonate compound which is produced is sodium bicarbonate; or the carbonate is potassium carbonate and the bicarbonate compound which is produced is potassium bicarbonate; or the carbonate is cesium carbonate and the bicarbonate compound which is produced is cesium bicarbonate; or the carbonate is ammonium carbonate and the bicarbonate compound which is produced is ammonium bicarbonate.

In another optional aspect, the aqueous solution is contacted with the CO₂-containing gas in a bubble column, a packed tower or a spray scrubber. In a preferred embodiment, the aqueous solution is contacted with the CO₂-containing gas in a bubble column.

In another optional aspect, the CO₂-containing gas comes from a power and/or steam plant flue gas, an industrial exhaust gas, or a chemical production flue gas. In one preferred embodiment, the CO₂-containing gas comes from a flue gas from a coal power and/or steam station, a flue gas from a gas power and/or steam station, a flue gas from metals production, an emission from lime kilns, a flue gas from a bicarbonate unit or a flue gas from a soda ash mill.

In another optional aspect, the CO₂-containing gas contains from 1 to 100 % (v/v) CO₂. In a preferred embodiment, the gas contains between 1 and 50 % (v/v) of CO₂.

In another optional aspect, the aqueous solution is contacted with the CO₂-containing gas at a temperature ranging from about 20°C to about 100°C.

In another optional aspect, the aqueous solution is contacted with the CO₂-containing gas at a CO₂ partial pressure ranging from about 1 to about 20 bars.

In another optional aspect, the carbonic anhydrase or analogue thereof is recycled to the step of contacting the aqueous solution with the CO₂-containing gas after the separation step.

In another optional aspect, the separation and conversion steps comprise precipitating the bicarbonate in the bicarbonate loaded stream and subjecting the same to thickening to produce the bicarbonate depleted stream as a thickener overflow including the carbonic anhydrase or analogue thereof and a thickener underflow including the bicarbonate compound. In one preferred embodiment, the thickening is performed in a hydrocyclone or a gravity separator.

In another optional aspect, the bicarbonate loaded stream is a solution without substantial precipitates when subjected to separation to remove the carbonic anhydrase or analogue thereof. In one preferred embodiment, the separation is performed in a filtration device. In another preferred embodiment, the solution without substantial precipitates is obtained by providing the solution at a sufficient temperature for solubility of the bicarbonate. Preferably, the sufficient temperature is achieved by heating the bicarbonate loaded stream. After separation of the carbonic anhydrase or analogue thereof, precipitated bicarbonate is preferably obtained by cooling the solution.

In another optional aspect, the precipitated bicarbonate is removed from the bicarbonate depleted stream in a thick slurry stream, and the process further includes dewatering the thick slurry stream in a spin dryer to produce a spin dried stream, and drying the spin dried stream in a flash dryer to produce the bicarbonate compound.

According to the invention, the precipitated bicarbonate is removed from the bicarbonate depleted stream in a thick slurry stream, and the process further includes drying the thick slurry stream to produce the bicarbonate compound.

According to the invention, the process further includes precipitating the bicarbonate so as to avoid substantial entrapment of the carbonic anhydrase or analogue thereof in the precipitated bicarbonate.

According to the invention, the process further includes precipitating the bicarbonate so as to avoid substantial adsorption of the carbonic anhydrase or analogue thereof in the precipitated bicarbonate.

An integrated process for production of a bicarbonate compound includes:
contacting a first aqueous solution including a dissolved carbonate with a CO₂-containing gas in the presence of carbonic anhydrase or an analogue thereof to produce an enzyme and bicarbonate loaded stream;
contacting a second solution including a dissolved carbonate with CO₂-containing gas to produce a bicarbonate loaded stream; wherein the carbonate in the second solution is the same than in the first aqueous solution;
combining the two streams to produce a loaded stream including carbonic anhydrase or analogue thereof and bicarbonate;
separating the carbonic anhydrase or analogue thereof from the loaded stream; and
converting the loaded stream into precipitated bicarbonate and a bicarbonate depleted stream, the precipitated bicarbonate including the bicarbonate compound.

In one optional aspect, the process produces a stable bicarbonate compound.

In another optional aspect, the carbonate is sodium carbonate and the bicarbonate compound which is produced is sodium bicarbonate; or the carbonate is potassium carbonate and the bicarbonate compound which is produced is potassium bicarbonate; or the carbonate is cesium carbonate and the bicarbonate compound which is produced is cesium bicarbonate; or the carbonate is ammonium carbonate and the bicarbonate compound which is produced is ammonium bicarbonate.

In another optional aspect, the aqueous solution is contacted with the CO₂-containing gas in a bubble column, a packed tower or a spray scrubber. In one preferred embodiment, the aqueous solution is contacted with the CO₂-containing gas in a bubble column.

In another optional aspect, the CO₂-containing gas comes from a power and/or steam plant flue gas, an industrial exhaust gas, or a chemical production flue gas. In one preferred embodiment, the CO₂-containing gas comes from a flue gas from a coal power and/or steam station, a flue gas from a gas power and/or steam station, a flue gas from metals production, an emission from lime kilns, a flue gas from a bicarbonate unit or a flue gas from a soda ash mill.

In another optional aspect, the CO₂-containing gas contains from 1 to 100 % (v/v) CO₂. In one preferred embodiment, the gas contains between 1 and 50 % (v/v) of CO₂.

In another optional aspect, the carbonic anhydrase is recycled to the step of contacting the aqueous solution with the CO₂-containing gas, after the separation step.

In another optional aspect, the separation and conversion steps comprise precipitating the bicarbonate in the bicarbonate loaded stream and subjecting the same to thickening to produce the bicarbonate depleted stream as a thickener overflow including the carbonic anhydrase or analogue thereof and a thickener underflow including the bicarbonate compound. In one embodiment, the thickening is performed in a hydrocyclone or a gravity separator.

In another optional aspect, the precipitated bicarbonate is removed from the bicarbonate depleted stream in a thick slurry stream, and the integrated process further includes dewatering the thick slurry stream in a spin dryer to produce a spin dried stream, and drying the spin dried stream in a flash dryer to produce the bicarbonate compound.

In another optional aspect, the precipitated bicarbonate is removed from the bicarbonate depleted stream in a thick slurry stream, and the integrated process further includes drying the thick slurry stream to produce the bicarbonate compound.

The invention according to claim 2 further provides an integrated process for production of a bicarbonate compound, including:
contacting a first aqueous solution including a dissolved carbonate and a carbonic anhydrase or analogue thereof with a CO₂-containing gas to produce an enzyme and bicarbonate loaded stream;
separating the carbonic anhydrase or analogue thereof from the bicarbonate loaded stream;
combining the bicarbonate loaded stream with a second solution including a dissolved carbonate, wherein the carbonate in the second solution is the same than in the first solution, to produce a bicarbonate and carbonate aqueous solution;
contacting the bicarbonate and carbonate aqueous solution with CO₂-containing gas to produce a second bicarbonate loaded stream;
converting the second bicarbonate loaded stream into precipitated bicarbonate and a bicarbonate depleted stream, the precipitated bicarbonate including the bicarbonate compound;
wherein the precipitated bicarbonate is removed from the bicarbonate depleted stream in a thick slurry stream; and
wherein the process further comprises drying the thickened slurry stream to produce the bicarbonate compound.

In one optional aspect, the process produces a stable bicarbonate compound.

In another optional aspect, the carbonate is sodium carbonate and the bicarbonate compound which is produced is sodium bicarbonate; or the carbonate is potassium carbonate and the bicarbonate compound which is produced is potassium bicarbonate; or the carbonate is cesium carbonate and the bicarbonate compound which is produced is cesium bicarbonate; or the carbonate is ammonium carbonate and the bicarbonate compound which is produced is ammonium bicarbonate.

In another optional aspect, the aqueous solution is contacted with the CO₂-containing gas in a bubble column, a packed tower or a spray scrubber. In one preferred embodiment, the aqueous solution is contacted with the CO₂-containing gas in a bubble column.

In another optional aspect, the CO₂-containing gas comes from a power and/or steam plant flue gas, an industrial exhaust gas, or a chemical production flue gas. In one preferred embodiment, the CO₂-containing gas comes from a flue gas from a coal power and/or steam station, a flue gas from a gas power and/or steam station, a flue gas from metals production, an emission from lime kilns, a flue gas from a bicarbonate unit or a flue gas from a soda ash mill.

In another optional aspect, the CO₂-containing gas contains from 1 to 100 % (v/v) CO₂. In one preferred embodiment, the gas contains between 1 and 50 % (v/v) of CO₂.

In another optional aspect, the bicarbonate loaded stream is a solution without substantial precipitates when subjected to separation to remove the carbonic anhydrase or analogue thereof. In one preferred embodiment, the separation is performed in a filtration device.

In another optional aspect, the carbonic anhydrase or analogue thereof is recycled to the step of contacting the aqueous solution with the CO₂-containing gas, after the separation step.

In another optional aspect, the conversion step comprises precipitating the bicarbonate in the second bicarbonate loaded stream and subjecting the same to thickening to produce the bicarbonate depleted stream as a thickener overflow and a thickener underflow including the bicarbonate compound. In one preferred embodiment, the thickening is performed in a hydrocyclone or a gravity separator.

In another optional aspect, the precipitated bicarbonate is removed from the bicarbonate depleted stream in a thick slurry stream, and the integrated process further includes dewatering the thick slurry stream in a spin dryer to produce a spin dried stream, and drying the spin dried stream in a flash dryer to produce the bicarbonate compound.

According to the invention, the precipitated bicarbonate is removed from the bicarbonate depleted stream in a thick slurry stream, and the integrated process further includes drying the thick slurry stream to produce the bicarbonate compound.

An enzymatic process for production of sodium bicarbonate comprises:
contacting an aqueous solution including dissolved sodium carbonate with a CO₂-containing gas in the presence of carbonic anhydrase or an analogue thereof to produce a sodium bicarbonate loaded stream;
separating the carbonic anhydrase or analogue thereof from the sodium bicarbonate loaded stream; and
converting the sodium bicarbonate loaded stream into precipitated sodium bicarbonate and a bicarbonate depleted stream.

Moreover, an integrated process for production of sodium bicarbonate comprises:
contacting an aqueous solution including dissolved sodium carbonate with a CO₂-containing gas in the presence of carbonic anhydrase or an analogue thereof to produce an enzyme and sodium bicarbonate loaded stream;
contacting a second solution including dissolved sodium carbonate with CO₂-containing gas to produce a sodium bicarbonate loaded stream;
combining the two streams to produce a loaded stream including carbonic anhydrase or analogue thereof and sodium bicarbonate;
separating the carbonic anhydrase or analogue thereof from the loaded stream; and
converting the loaded stream into precipitated sodium bicarbonate and a bicarbonate depleted stream.

An integrated process for production of sodium bicarbonate comprises:
contacting an aqueous solution including dissolved sodium carbonate with a CO₂-containing gas in the presence of carbonic anhydrase or analogue thereof to produce an enzyme and sodium bicarbonate loaded stream;
separating the carbonic anhydrase or analogue thereof from the sodium bicarbonate loaded stream;
combining the sodium bicarbonate stream with a second solution including dissolved sodium carbonate to produce a sodium bicarbonate and carbonate aqueous solution;
contacting the sodium bicarbonate and carbonate aqueous solution with CO₂-containing gas to produce a second sodium bicarbonate loaded stream;
converting the second sodium bicarbonate loaded stream into precipitated sodium bicarbonate and a bicarbonate depleted stream.

In another aspect, an enzyme enhanced process for the production of sodium bicarbonate from CO₂ includes a reaction with ammonia.

More particularly, an enzymatic process for production of sodium bicarbonate includes:
contacting an aqueous solution including dissolved ammonia with a CO₂-containing gas in the presence of carbonic anhydrase or analogue thereof to produce an enzyme and ammonium bicarbonate loaded stream;
separating the carbonic anhydrase or analogue thereof from the ammonium bicarbonate loaded stream; and
converting the ammonium bicarbonate loaded stream into precipitated sodium bicarbonate in the presence of sodium chloride.

In one optional aspect, the aqueous solution is contacted with the CO₂-containing gas in a bubble column.

In another optional aspect, the CO₂-containing gas comes from a power and/or steam plant flue gas, an industrial exhaust gas, or a chemical production flue gas. In one preferred embodiment, the CO₂-containing gas comes from a flue gas from a coal power and/or steam station, a flue gas from a gas power and/or steam station, a flue gas from metals production, an emission from lime kilns, a flue gas from a bicarbonate unit or a flue gas from a soda ash mill.

In another optional aspect, the CO₂-containing gas contains from 1 to 100 % (v/v) CO₂. In one preferred embodiment, the gas contains between 1 and 50 % (v/v) of CO₂.

In another optional aspect, the carbonic anhydrase or analogue thereof is recycled to the step of contacting the aqueous solution with the CO₂-containing gas after the separation step.

In another optional aspect, the process further includes a step of separating precipitated sodium bicarbonate by filtration. The process advantageously further includes dewatering precipitated sodium bicarbonate in a spin dryer and may further includes a step of drying sodium bicarbonate in a flash dryer.

While the invention will be described in conjunction with example embodiments, it will be understood that it is not intended to limit the scope of the invention to such embodiments. On the contrary, it is intended to cover all alternatives, modifications and equivalents as may be included as defined by the present description. The objects, advantages and other features of the present invention will become more apparent and be better understood upon reading of the following non-restrictive description of the invention, given with reference to the accompanying drawings.

### BRIEF DESCRIPTION OF THE DRAWINGS

Embodiments of the process according to the present invention are represented in the following figures.
Fig. 1 is a process diagram of an embodiment of the present invention.
Fig. 2 is a process diagram of another embodiment of the present invention.
Fig. 3 is a process diagram of a further embodiment of the present invention.
Fig. 4 is a process diagram of yet another embodiment of the present invention.
Fig. 5 is a scatter plot diagram representing the increase in CO₂ transfer rate using carbonic anhydrase in 0.3 M sodium carbonate based solutions.
Fig. 6 is a scatter plot diagram representing the impact of enzyme concentration and temperature on the increase in CO₂ absorption rate in 0.3 M Na₂CO₃ solution at a loading of 0.5 CO₂/mol Na⁺.

### DESCRIPTION OF PREFERRED EMBODIMENTS

In one aspect of the present invention, a bicarbonate compound is produced from the corresponding carbonate and CO₂ wherein the production of the bicarbonate is enhanced in the presence of at least one enzyme as a biocatalyst.

In one embodiment, the bicarbonate compound which is produced is a stable bicarbonate compound. A "stable bicarbonate compound" according to the invention is a bicarbonate compound that is stable in the solid state, as opposed, for example, to lithium bicarbonate which is stable in solution but not in the solid state. Examples of bicarbonate compounds which are stable in the solid state include sodium, potassium, cesium or ammonium bicarbonate. In a preferred embodiment, the process of the invention produces sodium bicarbonate.

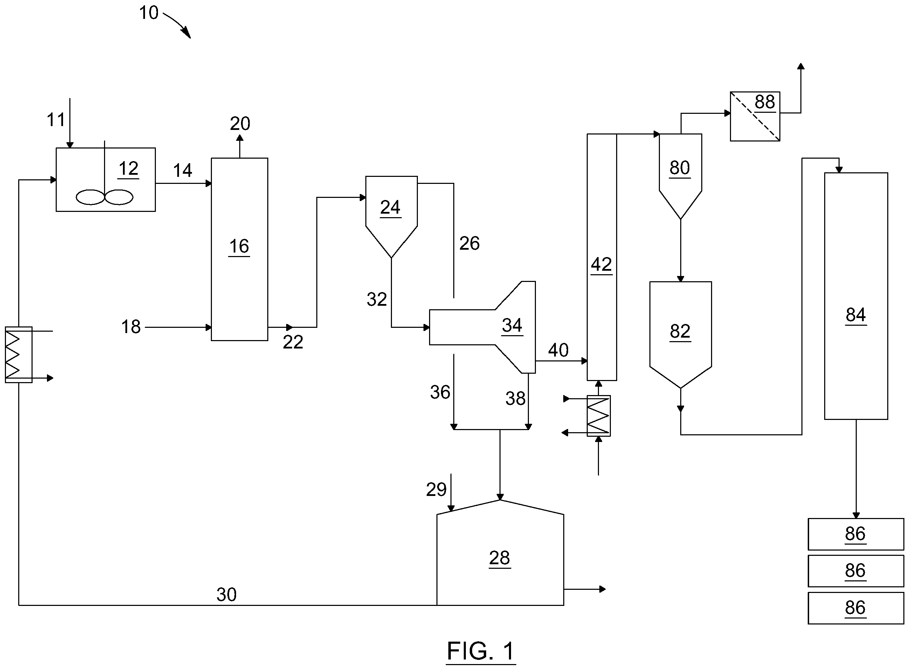

For clarity reasons, the following detailed description made in reference to Figs. 1-4, will refer to the production of sodium bicarbonate from sodium carbonate. However, the present invention also encompasses the production of any other bicarbonate from the corresponding carbonate. For example, the invention also concerns the production of potassium, cesium or ammonium bicarbonate from potassium, cesium or ammonium carbonate, respectively. Therefore, in the following description of the process of the present invention, one may use potassium carbonate (K₂CO₃), cesium carbonate (CS₂CO₃) or ammonium carbonate ((NH₄)₂CO₃) instead of sodium carbonate (Na₂CO₃) to produce potassium bicarbonate (KHCO₃), cesium bicarbonate (CsHCO₃) or ammonium bicarbonate (NH₄HCO₃), respectively.

Fig. 1 (according to the invention of claim 1) shows one implementation of a sodium bicarbonate production process **10.** A homogeneous sodium carbonate solution is prepared by dissolving solid Na₂CO₃ **11** in water in a dissolution unit **12.** The enzyme can be added to the sodium carbonate solution or may be already in the water prior to mixing with Na₂CO₃. The so obtained solution **14** is pumped to a reaction vessel **16** that may be a bubble column. The concentration of Na₂CO₃ in solution **14** can be adjusted by addition of water or solid Na₂CO₃ in the dissolution unit **12** during the process. Moreover, the concentration of Na₂CO₃ can be adjusted depending on the temperature of the solution **14** and/or the reaction temperature in reaction vessel **16.** Preferably, the concentration of Na₂CO₃ is up to the solubility limit for temperatures ranging from about 20°C to about 100°C. Solution **14** flows from the top to the bottom of the bubble column **16.** At the bottom of the column **16,** a CO₂-containing gas **18** is bubbled through the column which contains the sodium carbonate solution and the enzyme. The CO₂-containing gas **18** may contain from 1 to 100 % (v/v) CO₂. Preferably, the CO₂-containing gas **18** contains between 1 and 50 % (v/v). As the gas **18** flows through and contacts the solution **14,** CO₂ is absorbed and the CO₂ concentration in the gas decreases. The depleted CO₂ gas **20** exits the system at the top of the column **16.**

In the bubble column **16,** CO₂ is absorbed and reacts according to reaction 1, and also according to reaction 2, which corresponds to the CO₂ hydration reaction catalyzed by the enzyme, a carbonic anhydrase or analogue thereof.

CO₂ + OH⁻ ⇄ HCO₃⁻ Reaction 1

CO₂ + H₂O ⇄ HCO₃⁻ + H⁺. Reaction 2

As a result of both reactions taking place in the column, the fraction or amount of CO₂ absorbed by the solution increases compared to the case where no carbonic anhydrase is present in the reaction medium and only reaction 1 occurs. Consequently, the use of the carbonic anhydrase enhances CO₂ absorption and decreases or eliminates CO₂ slip. Furthermore, due to the fact that both reactions 1 and 2 take place in the bubble column, a higher bicarbonate ion concentration in the solution is obtained. Since the solution also contains sodium ions from the dissociation of sodium carbonate in water, a higher concentration of sodium bicarbonate is also obtained.

In one implementation, the reaction vessel **16** is a bubble column. In other implementation, the reaction vessel may be a direct-contact type reactor such as a packed tower or spray scrubber.

In the scenario in which the reaction vessel **16** is a packed tower, it may be sized, configured and operated such that the mass transfer and reaction rate are sufficient to reduce or eliminate CO₂ slip due to the enzymatic acceleration of CO₂ hydration. The conditions of the packed tower may be maintained to avoid precipitation in the reaction vessel itself.

The temperature at which CO₂ absorption is carried out in the reaction vessel **16** may range from about 20°C to about 100°C, or from about 20°C to 90 °C. The CO₂ partial pressure may range from about 1 to about 20 bars, or from about 1 to about 10 bars. The CO₂-containing gas **18** can come from a variety of sources. In one aspect, the CO₂-containing gas **18** is an effluent gas such as power and/or steam plant flue gas, industrial exhaust gas, or chemical production flue gas. It can be a raw flue gas from a coal power and/or steam station, flue gas from a gas power and/or steam station, flue gas from metals production (e.g. alumina and steel production), emission from lime kilns, etc. These flue gases may be pretreated so as to remove certain undesirable components such as NOₓ, SOₓ, and so on. In another optional aspect, the CO₂-containing gas **18** includes effluent gas from a bicarbonate unit or from a soda ash mill. It is worth noting that pure CO₂ gas may also be used in the process.

As shown in Fig. 1, an ion loaded stream **22,** which may be a slurry, exits the bubble column **16** at the bottom thereof. Slurry **22** which contains sodium bicarbonate both in solution and as solid particles and the enzyme is then fed to a thickener **24,** such as a hydrocyclone or gravity separator, to separate the aqueous solution containing the enzyme and increase solid particles concentration in the slurry. The solid particle concentration at the exit of the thickener may range from about 60 to about 95 % (w/w). The aqueous solution comprising the enzyme **26** exiting the thickener **24** is then pumped to a water tank or reservoir **28** wherein fresh water **29** may be added as required. The aqueous solution **30** in the reservoir **28,** which still contains the enzyme, may be recycled to the dissolution unit **12** for the preparation of the sodium carbonate solution **14** to be used in the bubble column. The enzyme concentration can be adjusted either in the dissolution unit **12,** or in another reservoir (not represented in the figures) downstream the reservoir **28.** Also, the temperature of the aqueous solution **30** may be adjusted as required before entering the dissolution unit **12.**

The slurry **32** leaving the thickener **24** is then sent to a spin dryer **34** for further dewatering. The percentage of solid after dewatering may be from about 95 to about 99 % (w/w). The liquid fraction **38** exiting the spin dryer is pumped to the reservoir **28.** In a preferred embodiment, the liquid fraction **38** is mixed with the aqueous solution **26** exiting the thickener before being delivered to the reservoir **28.** The wet solid fraction of sodium bicarbonate **40** exiting the spin dryer is then sent to a flash dryer **42** to complete drying of the particles until a target water content is reached. Preferably, the solid exiting the flash dryer **42** is further sent to a cyclone **80** before being conveyed to a silo **82** wherein it can be stocked. The overflow exiting the cyclone **80** may be filtered through a filter **88.** The sodium bicarbonate stocked in the silo **82** may further be sent to delivery silos **84.** From there, bags **86** of sodium bicarbonate can be prepared for shipment to customers.

In general, the process takes advantage of an enzyme as a biocatalyst for enhancing sodium bicarbonate production from CO₂ and sodium carbonate. As previously mentioned, the biocatalyst may be at least one enzyme such as carbonic anhydrase or analogue thereof. The carbonic anhydrase may be from human, bacterial, fungal or other organism origins, having thermostable or other stability properties, as long as the carbonic anhydrase or analogue thereof can catalyze the hydration of the carbon dioxide to form hydrogen and bicarbonate (see Reaction 2 above). It should also be noted that "carbonic anhydrase or an analogue thereof as used herein includes naturally occurring, modified, recombinant and/or synthetic enzymes including chemically modified enzymes, enzyme aggregates, cross-linked enzymes, enzyme particles, enzyme-polymer complexes, polypeptide fragments, enzyme-like chemicals such as small molecules mimicking the active site of carbonic anhydrase enzymes and any other functional analogue of the enzyme carbonic anhydrase.

The enzyme carbonic anhydrase may have a molecular weight up to about 100,000 daltons. In another embodiment, the carbonic anhydrase can be of relatively low molecular weight (e.g. 30,000 daltons).

The carbonic anhydrase or analogue thereof may be provided in various ways. It may be supported on or in particles that flow with the solution, directly bonded to the surface of particles, entrapped inside or fixed to a porous support material matrix, entrapped inside or fixed to a porous coating material that is provided around a support particle that is itself porous or non-porous, or present as cross linked enzyme aggregates (CLEA) or cross linked enzyme crystals (CLEC). The enzymes may be provided immobilized within the reactor itself, or may be free in the solution.

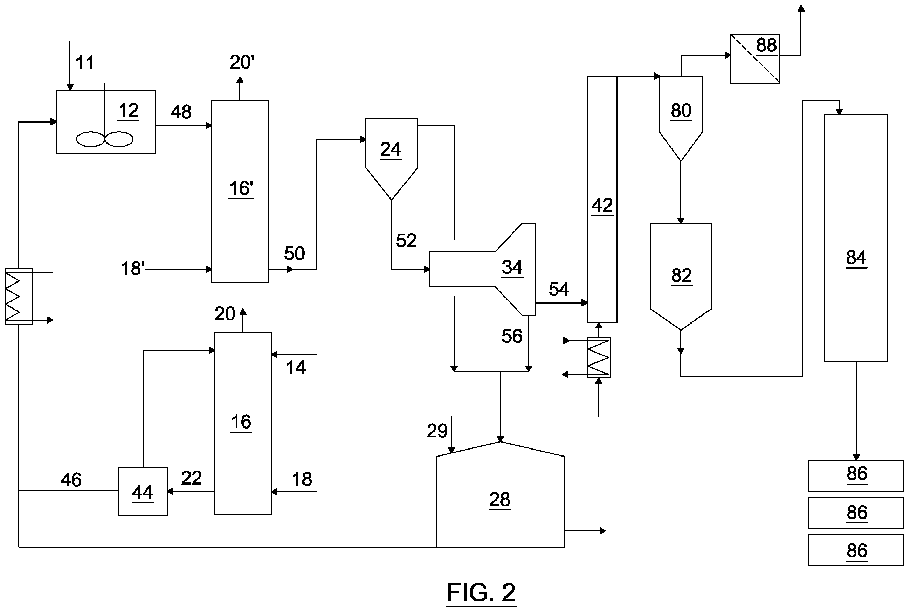

Fig. 2 (according to the invention of claim 2) shows another implementation of the process of the present invention. In this implementation, the process has an upstream enzymatic system and a downstream system for producing sodium bicarbonate which may not use a biocatalyst. As seen in Fig. 2, a Na₂CO₃ aqueous solution containing carbonic anhydrase **14** is prepared and then fed to a first bubble column **16.** The solution 14 is allowed to flow from the top to the bottom of the bubble column, while a CO₂ containing gas **18** is concomitantly bubbled through the column, from the bottom thereof. As the gas **18** flows through the solution **14,** CO₂ is absorbed according to Reactions 1 and 2 mentioned above. Then, CO₂ concentration in the gas decreases while bicarbonate ions concentration in solution increases. In this particular embodiment, the absorption conditions are adjusted in such a way that the bicarbonate concentration is low enough to avoid any sodium bicarbonate precipitation. For example, sodium bicarbonate precipitation is avoided by controlling the concentration of bicarbonate ions in solution and/or the reaction temperature. In this particular embodiment, the stream **22** exiting the reactor does not contain any solid particles and is a homogenous solution. This is different than the slurry **22** exiting the reactor according to the first implementation of the process as explained above, which contains both sodium bicarbonate in solution and as solid particles.

Then, the CO₂-depleted gas **20** exits the system at the top of the column, and the sodium bicarbonate solution containing the enzyme **22** is pumped to a separator **44.** The separator **44** removes the enzyme from the solution **22** and the enzyme rich solution is sent back to the bubble column. The separator may include a filtration device, for example a membrane filtration. The enzyme free solution **46** exiting the separator and which contains sodium bicarbonate in solution, is then pumped to a dissolution unit **12** where additional solid Na₂CO₃ **11** is dissolved in the solution. Solution **48** containing both sodium carbonate and sodium bicarbonate in solution exits the dissolution unit **12** and is then fed to the reaction vessel **16',** for example a bubble column, where it is contacted to a CO₂-containing gas **18'.** The CO₂-containing gas **18'** may come from the same source than the CO₂-containing gas **18,** or from a different source. In reactor **16',** Reaction 1 mentioned above occurs and the concentration in bicarbonate ions increases. The CO₂-depleted gas **20'** exits at the top of the column **16'.** The slurry **50** containing sodium bicarbonate both in solution and as solid particles exiting the bubble column, is then pumped to a thickener **24** to remove excess water which is pumped to a reservoir **28.**

The slurry **52** exiting the thickener is then fed to a spin dryer **34** where it is dewatered until it reaches a water content of about 2% (w/w). The wet solid **54** exiting the spin dryer is then sent to a flash dryer **42** to complete drying of the particles, while the solution **56** containing mostly water is sent to the reservoir **28.** The solution in the reservoir can be recycled in the process and more particularly to the dissolution unit **12.** In a preferred embodiment, the water exiting the reservoir **28** is mixed with the sodium bicarbonate solution **46** exiting the separator **44** before to reach the dissolution unit **12.** As explained above for the first implementation of the process, the solid exiting the flash dryer may further be sent to a cyclone before being conveyed to a silo wherein it can be stocked or further sent to delivery silos. From there, bags of sodium bicarbonate can be prepared for shipment to the customers.

It is worth mentioning that in this particular embodiment, as shown in Fig. 2, the reservoir **28** contains mainly water. This differs from the first embodiment shown in Fig. 1 wherein the water solution in reservoir **28** also comprises the enzyme. In the process of Fig. 2, the enzyme is "confined" in the upstream part of the process.

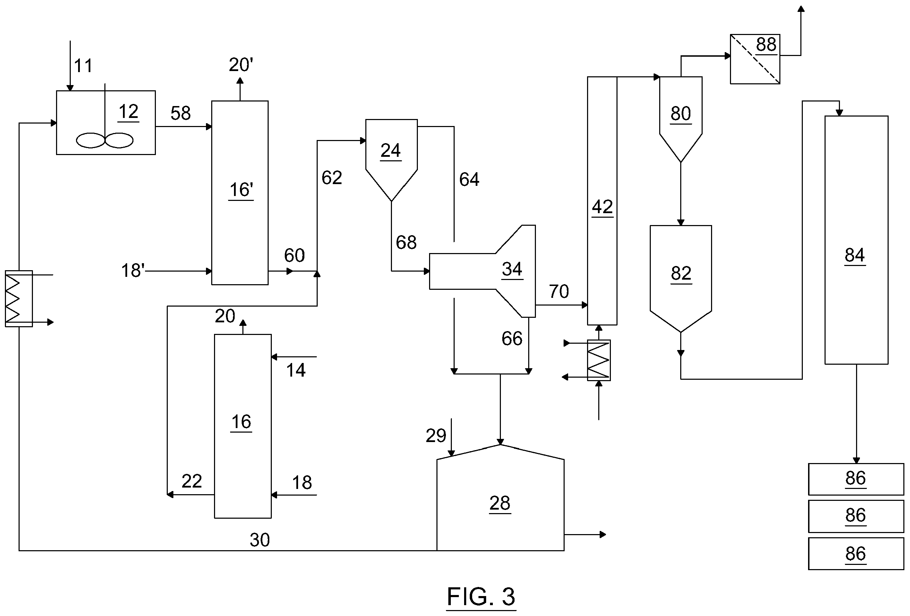

Fig. 3 (comparative) represents another implementation of a process. While the overall process shown in Fig. 3 combines a process with a system for producing sodium bicarbonate which does not use a biocatalyst as in Fig. 2, it however differs from the process of Fig. 2 in that the enzymatic system is operated in parallel with the system which does not use a biocatalyst. In the process of Fig. 2, the two systems are in series.

In this implementation of Fig. 3, the enzyme is first present in a sodium carbonate solution and the obtained solution **14** is fed to the bubble column **16.** A CO₂-containing gas **18** is sent to the bubble column and is allowed to flow through the solution **14** containing the enzyme and CO₂ is absorbed according to Reactions 1 and 2. The conditions are adjusted in such a way that the bicarbonate concentration level is kept low enough to avoid precipitation of sodium bicarbonate. The CO₂-depleted gas **20** exits at the top of the column. In parallel, a solution of sodium carbonate **58** which has been prepared in a dissolution unit **12,** is fed to a second bubble column **16'** wherein it comes into contact with a CO₂-containing gas **18'.** CO₂ absorbs into the solution **58** according to Reaction 1 and the concentration in bicarbonate ions increases. The slurry **60** containing sodium bicarbonate both in solution and as solid particles exiting the bubble column **16',** is then mixed with the homogeneous solution **22** containing the enzyme and the sodium bicarbonate obtained in the first bubble column **16.** The combined slurry **62** is then pumped to a thickener **24** to separate the aqueous solution containing the enzyme and increase solid particles concentration in the slurry. From there, the following steps are similar to the ones of the process represented in Fig. 1. The aqueous solution comprising the enzyme **64** exiting the thickener is pumped to a water tank or reservoir **28.** The slurry **68** leaving the thickener is then sent to a spin dryer **34** for further dewatering. The liquid fraction **66** exiting the spin dryer is pumped to the reservoir **28.** In a preferred embodiment, the liquid fraction **66** is mixed with the aqueous solution **64** exiting the thickener before to be delivered to the water tank **28.** The wet solid fraction of sodium bicarbonate **70** exiting the spin dryer is then sent to a flash dryer **42** to complete drying of the particles until a target water content is reached. Preferably, the solid exiting the flash dryer is further sent to a cyclone before being conveyed to a silo wherein it can be stocked or further sent to delivery silos. From there, bags of sodium bicarbonate can be prepared for shipment to the customers. The aqueous solution containing the enzyme is recycled to form part of the solution **14** fed to the enzymatic reaction vessel **16.**

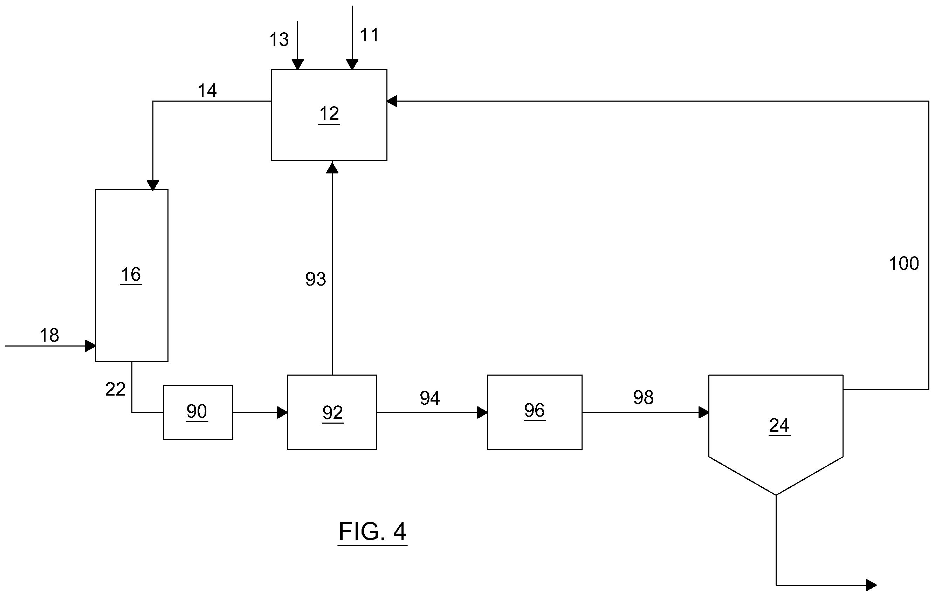

Fig. 4 (according to the invention of claim 1) represents a further implementation of the process. In this implementation, a homogenous aqueous solution **14** is prepared by dissolving the enzyme **13** and sodium carbonate **11** in water in dissolution unit **12.** The aqueous solution **14** is then fed to the reaction vessel **16,** *e.g.* a bubble column. The CO₂-containing gas **18** is sent to the bubble column and is allowed to flow through the solution **14** containing the enzyme, and CO₂ is absorbed according to Reactions 1 and 2. The stream **22** exiting the bubble column then goes through a heat exchanger **90** wherein the stream is heated to avoid solid particles precipitation. The solution exiting the heat exchanger **90,** which contains the enzyme, is then sent to a separator **92** such as a filtration device, to remove the enzyme. The enzyme rich stream **93** recovered from the separator **92** is recycled to the dissolution unit **12.** The enzyme free sodium bicarbonate solution **94** exiting the separator is sent to a heat exchanger **96** wherein it is cooled to allow sodium bicarbonate precipitation. The so obtained slurry **98** is then pumped to a thickener **24** to increase solid particles concentration in the slurry. The aqueous stream depleted of sodium bicarbonate **100** exiting the thickener **24** is then recycled to the dissolution unit **12.** The production of solid sodium bicarbonate from the slurry exiting the thickener is then carried out in the same way as described above. The slurry leaving the thickener may be sent to a spin dryer for further dewatering and the obtained wet solid fraction may be further sent to a flash dryer to complete drying of the particles until a target water content is reached. Preferably, the solid exiting the flash dryer is further sent to a cyclone before being conveyed to a silo wherein it can be stocked or further sent to delivery silos. From there, bags of sodium bicarbonate can be prepared for shipment to the customers. In a further process, sodium bicarbonate is produced according to a process involving a reaction between ammonia and CO₂, which is catalyzed by the enzyme carbonic anhydrase.

In a first step of this particular process, CO₂ is absorbed by an ammonia (NH₃) based solution, thereby forming ammonium bicarbonate [NH₄⁺; HCO₃⁻] in aqueous solution, according to Reaction 3 as follows:

CO₂ + H₂O + NH₃ → NH₄HCO₃ Reaction 3.

The formation of ammonium bicarbonate in solution involves the hydration of gaseous CO₂ into bicarbonate ions (HCO₃⁻) and hydrogen ions (H⁺), which may be catalyzed by the enzyme carbonic anhydrase. In aqueous solution, Reaction 3 may therefore be written as follows:

HCO₃⁻ + H⁺ + NH₃ → NH₄⁺ + HCO₃⁻.

In a second step of this process, ammonium bicarbonate reacts with NaCI to form sodium bicarbonate as follows:

NH₄HCO₃ + NaCl → NaHCO₃ + NH₄Cl

In an implementation of this process, an ammonia absorption solution including the enzyme is first fed to a reaction vessel wherein it comes into contact with a CO₂-containing gas. CO₂ dissolves into the absorption solution and hydration of dissolved CO₂ into bicarbonate ions and hydrogen ions is catalyzed in presence of the enzyme. The CO₂-depleted gas exits the bubble column at the top thereof while an ion-rich solution comprising bicarbonate ions and ammonium ions exits the column at the bottom thereof. In a preferred embodiment the enzyme is separated from the ion-rich solution comprising bicarbonate ions and ammonium ions before reacting the ion-rich solution with NaCl to produce sodium bicarbonate. Sodium bicarbonate appears as a solid since it is not soluble in ammonium chloride solution and it can be separated from the solution by filtration. From there, the solid can be further sent to a flash dryer to complete drying of the particles until a target water content is reached. Preferably, the solid exiting the flash dryer is further sent to a cyclone before being conveyed to a silo wherein it can be stocked or further sent to delivery silos. From there, bags of sodium bicarbonate can be prepared for shipment to the customers.

In another embodiment of this latest process, sodium bicarbonate obtained by filtration is heated to form sodium carbonate (Na₂CO₃). For example, heating is performed in a calciner at about 160-230 °C. The CO₂ which is released by heating sodium bicarbonate may be recycled back into the process.

### EXAMPLES

### Example 1: Impact of enzyme in 0.3 M Na₂CO₃ with CO₂ loading varying from 0.5 to 1 mol CO₂/mol Na⁺

The impact of carbonic anhydrase was determined by conducting experiments in stirred cell reactor at 25°C. An enzyme concentration varying from 0 to 1.6 g/L was added to 500 mL of a 0.3 M Na₂CO₃ having a CO₂ loading ranging from 0.5 to 1 mol CO₂/mol Na⁺ (a loading of 0.5 corresponds to a Na₂CO₃ solution and a loading of 1 corresponds to a NaHCO₃ solution, intermediate loadings correspond to mixtures of sodium carbonate/bicarbonate solutions). Enzyme used was supplied by Codexis inc. After applying vacuum to the solution, pure CO₂ is injected into the reactor up to an initial pressure comprised between 30 and 300 mbar. Then CO₂ pressure is monitored overtime. From these data, a mean CO₂ transfer (absorption) rate corresponding to a 90% CO₂ pressure decrease is calculated for the system with and without enzyme (for a CO₂ loading of 1 mol/mol, pressure decrease was 56%). Increase in CO₂ transfer rate is calculated by dividing the CO₂ transfer rate with the enzyme by the CO₂ transfer rate without the enzyme.

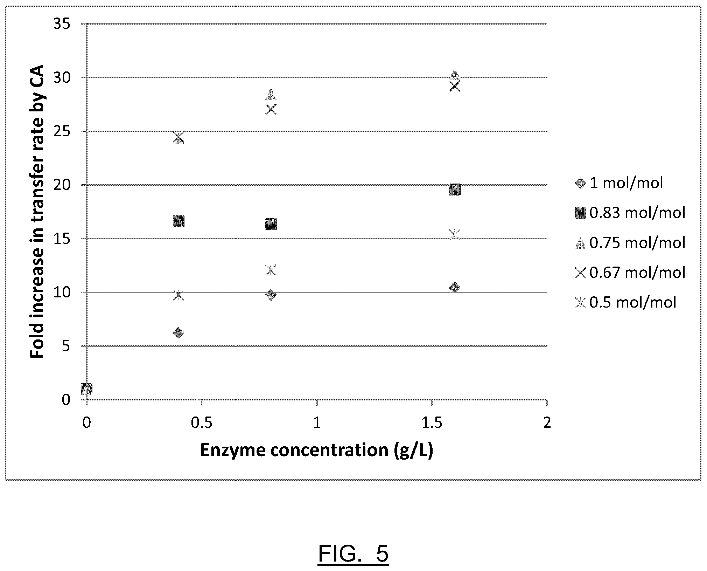

Results are reported in Figure 5. These results show that adding carbonic anhydrase to the solution increases the CO₂ absorption rate and that the increase is related to the enzyme concentration in the solution. Moreover it can be observed that the increase is also related to the CO₂ loading values.

### Example 2: Impact of enzyme concentration and temperature on the CO₂ absorption rates in 0.3 M Na₂CO₃ at a CO₂ loading of 0.5 mol CO₂/mol Na⁺

The impact of carbonic anhydrase concentration and solution temperature was determined by conducting experiments in stirred cell reactor at 25, 40 and 60°C. An enzyme concentration varying from 0 to 2.4 g/L was added to 500 mL of a 0.3 M Na₂CO₃ having a loading of 0.5 mol CO₂/mol Na⁺. Enzyme was supplied by Codexis inc. After applying vacuum to the solution, pure CO₂ is injected into the reactor up to an initial pressure comprised between 30 and 300 mbar). Then CO₂ pressure is monitored overtime. From these data, a mean CO₂ transfer (absorption) rate corresponding to a 90% CO₂ pressure decrease is calculated for the system with and without enzyme. Increase in CO₂ transfer rate is calculated by dividing the CO₂ transfer rate with the enzyme by the CO₂ transfer rate without the enzyme.

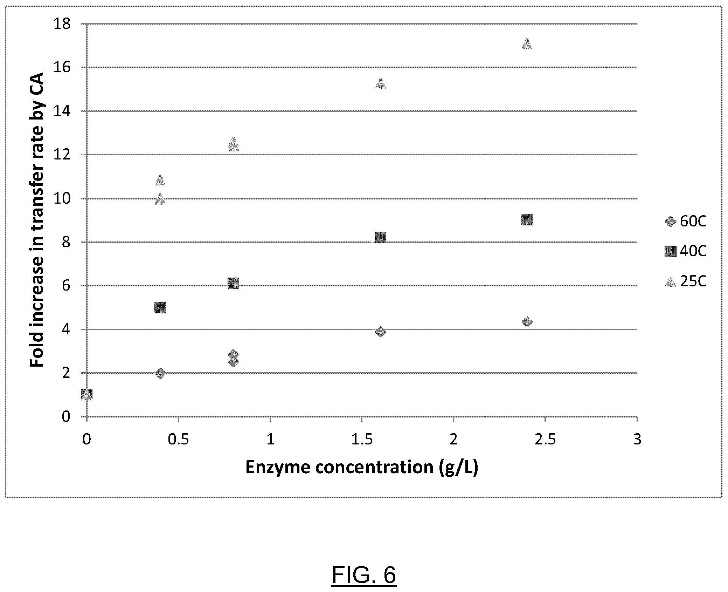

Results are reported in Figure 6. These results show that the enzyme concentration and temperature have an impact on the CO₂ absorption rates. As previously mentioned, increase in enzyme concentration leads to an increase in CO₂ absorption rates. Regarding temperature, impact of the enzyme is higher at lower temperature where the solvent contribution to CO₂ absorption is smaller.

### Example 3: Impact of enzyme in 1.45 M K₂CO₃ with CO₂ loadings of 0.5 and 0.6 mol CO₂/mol K⁺

The impact of carbonic anhydrase was determined by conducting experiments in stirred cell reactor at 25°C. An enzyme concentration of 0.2 g/L was added to 100 mL of a 1.45 M K₂CO₃ having a loading of 0.5 or 0.6 CO₂/mol K⁺ (a loading of 0.5 corresponds to a K₂CO₃ solution and a loading of 0.6 corresponds to a mixture of potassium carbonate/bicarbonate). Enzyme was supplied by Codexis inc. After applying vacuum to the solution, pure CO₂ is injected into the reactor up to an initial pressure of 1000 mbar. Then CO₂ pressure is monitored overtime. From these data, a mean CO₂ transfer (absorption) rate corresponding to a 90% CO₂ pressure decrease is calculated for the system with and without enzyme. Increase in CO₂ transfer rate is calculated by dividing the CO₂ transfer rate with the enzyme by the CO₂ transfer rate without the enzyme. Results are shown in the table below.

| **Solution** | **CO₂ loading (mol CO₂/mol K⁺)** | **Increase in transfer rate** |
|---|---|---|
| 1.45 M K₂CO₃ | 0.5 | 13.6 |
| 1.45 M K₂CO₃ | 0.6 | 22.1 |

These data show that carbonic anhydrase increases the CO₂ absorption rate in potassium carbonate solutions.

Although preferred embodiments of the present invention have been described herein in detail and illustrated in the accompanying drawings and examples, it is to be understood that the invention is not limited to these specific embodiments and that various changes and modifications may be effected thereto without departing from the scope of the present invention.

## Claims

1. An enzymatic process for production of a bicarbonate compound, comprising:
contacting an aqueous solution including a dissolved carbonate and a carbonic anhydrase or an analogue thereof with a CO₂-containing gas to produce an enzyme and bicarbonate loaded stream;
separating the carbonic anhydrase or analogue thereof from the bicarbonate loaded stream;
converting the bicarbonate loaded stream into precipitated bicarbonate and a bicarbonate depleted stream, the precipitated bicarbonate including the bicarbonate compound;
wherein the precipitated bicarbonate is removed from the bicarbonate depleted stream in a thick slurry stream; and
wherein the process further comprises drying the thickened slurry stream to produce the bicarbonate compound.

2. An integrated process for production of a bicarbonate compound, comprising:
contacting a first aqueous solution including a dissolved carbonate and a carbonic anhydrase or analogue thereof with a CO₂-containing gas to produce an enzyme and bicarbonate loaded stream;
separating the carbonic anhydrase or analogue thereof from the bicarbonate loaded stream;
combining the bicarbonate loaded stream with a second solution including a dissolved carbonate, wherein the carbonate in the second solution is the same than in the first solution, to produce a bicarbonate and carbonate aqueous solution;
contacting the bicarbonate and carbonate aqueous solution with CO₂-containing gas to produce a second bicarbonate loaded stream; and
converting the second bicarbonate loaded stream into precipitated bicarbonate and a bicarbonate depleted stream, the precipitated bicarbonate including the bicarbonate compound; and
wherein the precipitated bicarbonate is removed from the bicarbonate depleted stream in a thick slurry stream; and
wherein the process further comprises drying the thickened slurry stream to produce the bicarbonate compound.

3. The process of claim 1 or 2, wherein the aqueous solution is contacted with the CO₂-containing gas in a bubble column, a packed tower or a spray scrubber.

4. The process of claim 1 or 2, wherein the aqueous solution is contacted with the CO₂-containing gas in a bubble column.

5. The process of any one of claims 1 to 4, wherein the CO₂-containing gas comes from a power and/or steam plant flue gas, an industrial exhaust gas, or a chemical production flue gas, optionally from a flue gas from a coal power and/or steam station, a flue gas from a gas power and/or steam station, a flue gas from metals production, an emission from lime kilns, a flue gas from a bicarbonate unit or a flue gas from a soda ash mill.

6. The process of any one of claims 1 to 5, wherein the CO₂-containing gas contains from 1 to 100 % (v/v) of CO₂, optionally between 1 and 50 % (v/v) of CO₂.

7. The process of claim 1, wherein the aqueous solution is contacted with the CO₂-containing gas at a temperature ranging from 20°C to 100°C and/or at a CO₂ partial pressure ranging from 1 to 20 bars.

8. The process of any one of claims 1 to 7, wherein, after separation, the carbonic anhydrase or analogue thereof is recycled to the step of contacting the aqueous solution with the CO₂-containing gas.

9. The process of claim 1, wherein the bicarbonate loaded stream is a solution without substantial precipitates when subjected to separation to remove the carbonic anhydrase or analogue thereof.

10. The process of claim 9, wherein the solution without substantial precipitates is obtained by providing the solution at a sufficient temperature for solubility of the bicarbonate.

11. The process of claim 10, wherein the sufficient temperature is achieved by heating the bicarbonate loaded stream.

12. The process of claim 1, wherein the bicarbonate loaded stream is a solution without substantial precipitates, obtained by providing the solution at a sufficient temperature for solubility of the bicarbonate when subjected to separation to remove the carbonic anhydrase or analogue thereof and wherein, after separation of the carbonic anhydrase or analogue thereof, precipitated bicarbonate is obtained by cooling the solution.

13. The process of claim 1, further comprising precipitating the bicarbonate so as to avoid substantial entrapment and/or substantial adsorption of the carbonic anhydrase or analogue thereof in the precipitated bicarbonate.

14. The process of any one of claims 1 to 13, wherein the carbonate is sodium, potassium, cesium or ammonium carbonate, and the bicarbonate compound is a sodium, potassium, cesium or ammonium bicarbonate.

15. The process of any one of claims 1 to 13, wherein the carbonate is sodium carbonate and the bicarbonate compound is sodium bicarbonate.

## Patentansprüche

1. Enzymatisches Verfahren zur Herstellung einer Bicarbonatverbindung, umfassend:
Inkontaktbringen einer wässrigen Lösung, die ein gelöstes Carbonat und eine Carboanhydrase oder ein Analogon davon einschließt, mit einem CO₂-haltigen Gas, um einen mit Enzym und Bicarbonat beladenen Strom herzustellen;
Trennen der Carboanhydrase oder des Analogons davon vom mit Bicarbonat beladenen Strom;
Umwandeln des mit Bicarbonat beladenen Stroms in gefälltes Bicarbonat und einen bicarbonatarmen Strom, wobei das gefällte Bicarbonat die Bicarbonatverbindung einschließt;
wobei das gefällte Bicarbonat aus dem bicarbonatarmen Strom in einem Dickschlammstrom entfernt wird; und
wobei das Verfahren des Weiteren Trocknen des Dickschlammstroms umfasst, um die Bicarbonatverbindung herzustellen.

2. Integriertes Verfahren zur Herstellung einer Bicarbonatverbindung, umfassend:
Inkontaktbringen einer ersten wässrigen Lösung, die ein gelöstes Carbonat und eine Carboanhydrase oder ein Analogon davon einschließt, mit einem CO₂-haltigen Gas, um einen mit Enzym und Bicarbonat beladenen Strom herzustellen;
Trennen der Carboanhydrase oder des Analogons davon vom mit Bicarbonat beladenen Strom;
Kombinieren des mit Bicarbonat beladenen Stroms mit einer zweiten Lösung, die ein gelöstes Carbonat einschließt, wobei das Carbonat in der zweiten Lösung das gleiche ist wie in der ersten Lösung, um eine wässrige Bicarbonat- und Carbonatlösung herzustellen;
Inkontaktbringen der wässrigen Bicarbonat- und Carbonatlösung mit CO₂-haltigem Gas, um einen zweiten mit Bicarbonat beladenen Strom herzustellen; und
Umwandeln des zweiten mit Bicarbonat beladenen Stroms in gefälltes Bicarbonat und einen bicarbonatarmen Strom, wobei das gefällte Bicarbonat die Bicarbonatverbindung einschließt; und
wobei das gefällte Bicarbonat aus dem bicarbonatarmen Strom in einem Dickschlammstrom entfernt wird; und
wobei das Verfahren des Weiteren Trocknen des Dickschlammstroms umfasst, um die Bicarbonatverbindung herzustellen.

3. Verfahren nach Anspruch 1 oder 2, wobei die wässrige Lösung mit dem CO₂-haltigen Gas in einer Blasensäule, einer Füllkörpersäule oder einem Spritzwäscher in Kontakt gebracht wird.

4. Verfahren nach Anspruch 1 oder 2, wobei die wässrige Lösung mit dem CO₂-haltigen Gas in einer Blasensäule in Kontakt gebracht wird.

5. Verfahren nach einem beliebigen der Ansprüche 1 bis 4, wobei das CO₂-haltige Gas aus einem Kraftwerk- und/oder Dampfkraftwerkabgas, einem industriellen Abgas oder einem chemischen Produktionsabgas kommt, gegebenenfalls aus einem Abgas aus einem Kohlekraftwerk und/oder Dampfkraftwerk, einem Abgas aus einem Gaskraftwerk und/oder Dampfkraftwerk, einem Abgas aus der Metallproduktion, einer Emission von Kalkbrennereien, einem Abgas aus einem Bicarbonatbetrieb oder einem Abgas aus einem Sodawerk.

6. Verfahren nach einem beliebigen der Ansprüche 1 bis 5, wobei das CO₂-haltige Gas von 1 bis 100% (Vol./Vol.) CO₂, gegebenenfalls zwischen 1 und 50% (Vol./Vol.) CO₂ enthält.

7. Verfahren nach Anspruch 1, wobei die wässrige Lösung mit dem CO₂-haltigen Gas bei einer Temperatur im Bereich von 20°C bis 100°C und/oder bei einem CO₂-Partialdruck im Bereich von 1 bis 20 bar in Kontakt gebracht wird.

8. Verfahren nach einem beliebigen der Ansprüche 1 bis 7, wobei nach der Trennung die Carboanhydrase oder das Analogon davon zum Schritt des Inkontaktbringens der wässrigen Lösung mit dem CO₂-haltigen Gas rezirkuliert wird.

9. Verfahren nach Anspruch 1, wobei der mit Bicarbonat beladene Strom eine Lösung ohne wesentliche Präzipitate ist, wenn er einer Trennung unterzogen wird, um die Carboanhydrase oder das Analogon davon zu entfernen.

10. Verfahren nach Anspruch 9, wobei die Lösung ohne wesentliche Präzipitate durch Bereitstellen der Lösung bei einer ausreichenden Temperatur für die Löslichkeit des Bicarbonats erhalten wird.

11. Verfahren nach Anspruch 10, wobei die ausreichende Temperatur durch Erhitzen des mit Bicarbonat beladenen Stroms erreicht wird.

12. Verfahren nach Anspruch 1, wobei der mit Bicarbonat beladene Strom eine Lösung ohne wesentliche Präzipitate ist, der durch Bereitstellen der Lösung bei einer ausreichenden Temperatur für die Löslichkeit des Bicarbonats erhalten wird, wenn er einer Trennung unterzogen wird, um die Carboanhydrase oder das Analogon davon zu entfernen, und wobei nach Trennen der Carboanhydrase oder des Analogons davon gefälltes Bicarbonat durch Abkühlen der Lösung erhalten wird.

13. Verfahren nach Anspruch 1, das des Weiteren Fällen des Bicarbonats umfasst, um wesentlichen Einschluss und/oder wesentliche Adsorption der Carboanhydrase oder des Analogons davon im gefällten Bicarbonat zu vermeiden.

14. Verfahren nach einem beliebigen der Ansprüche 1 bis 13, wobei das Carbonat Natrium-, Kalium-, Cäsium- oder Ammoniumcarbonat ist, und die Bicarbonatverbindung ein Natrium-, Kalium-, Cäsium- oder Ammoniumbicarbonat ist.

15. Verfahren nach einem beliebigen der Ansprüche 1 bis 13, wobei das Carbonat Natriumcarbonat ist und die Bicarbonatverbindung Natriumbicarbonat ist.

## Revendications

1. Procédé enzymatique pour la production d'un composé bicarbonate, comprenant:
la mise en contact d'une solution aqueuse contenant un carbonate dissout et une anhydrase carbonique ou un analogue de celle-ci avec un gaz contenant du CO₂ pour produire un flux chargé d'enzyme et de bicarbonate;
la séparation de l'anhydrase carbonique ou de l'analogue de celle-ci du flux chargé de bicarbonate;
la conversion du flux chargé de bicarbonate en bicarbonate précipité et un flux appauvri en bicarbonate, le bicarbonate précipité contenant le composé bicarbonate;
dans lequel le bicarbonate précipité est retiré du flux appauvri en bicarbonate dans un flux d'une pâte épaisse; et
où le procédé comprend en outre le séchage du flux de pâte épaisse pour produire le composé bicarbonate.

2. Procédé intégré pour la production d'un composé bicarbonate, comprenant:
la mise en contact d'une première solution aqueuse contenant un carbonate dissout et une anhydrase carbonique ou un analogue de celle-ci avec un gaz contenant du CO₂ pour produire un flux chargé d'enzyme et de bicarbonate;
la séparation de l'anhydrase carbonique ou de l'analogue de celle-ci du flux chargé de bicarbonate;
la combinaison du flux chargé de bicarbonate avec une deuxième solution contenant un carbonate dissout, où le carbonate dans la deuxième solution est le même que dans la première solution, pour produire une solution aqueuse de bicarbonate et de carbonate;
la mise en contact de la solution aqueuse de bicarbonate et de carbonate avec un gaz contenant du CO₂ pour produire un deuxième flux chargé de bicarbonate; et
la conversion du deuxième flux chargé de bicarbonate en bicarbonate précipité et un flux appauvri en bicarbonate, le bicarbonate précipité contenant le composé bicarbonate;
dans lequel le bicarbonate précipité est retiré du flux appauvri en bicarbonate dans un flux d'une pâte épaisse; et
où le procédé comprend en outre le séchage du flux de pâte épaisse pour produire le composé bicarbonate.

3. Le procédé de la revendication 1 ou 2, dans lequel la solution aqueuse est mise en contact avec le gaz contenant du CO₂ dans une colonne à bulles, une tour à garnissage ou un épurateur à pulvérisation.

4. Le procédé de la revendication 1 ou 2, dans lequel la solution aqueuse est mise en contact avec le gaz contenant du CO₂ dans une colonne à bulles.

5. Le procédé de l'une quelconque des revendications 1 à 4, dans lequel le gaz contenant du CO₂ provient d'un gaz de combustion d'une centrale électrique et/ou à vapeur, d'un gaz d'échappement industriel, ou d'un gaz de combustion d'un procédé chimique, éventuellement d'un gaz de combustion d'une centrale à charbon et/ou vapeur, un gaz de combustion d'une centrale à gaz et/ou vapeur, un gaz de combustion de production de métaux, une émission de fours à chaux, un gaz de combustion d'une unité à bicarbonate ou un gaz de combustion d'une fabrique à soude.

6. Le procédé de l'une quelconque des revendications 1 à 5, dans lequel le gaz contenant du CO₂ contient de 1 à 100 % (v/v) de CO₂, éventuellement entre 1 et 50 % (v/v) de CO₂.

7. Le procédé de la revendication 1, dans lequel la solution aqueuse est mise en contact avec le gaz contenant du CO₂ à une température allant de 20°C à 100°C et/ou à une pression partielle de CO₂ allant de 1 à 20 bars.

8. Le procédé de l'une quelconque des revendications 1 à 7, dans lequel, après la séparation, l'anhydrase carbonique ou l'analogue de celle-ci est recyclée dans l'étape de mise en contact de la solution aqueuse avec le gaz contenant du CO₂.

9. Le procédé de la revendication 1, dans lequel le flux chargé de bicarbonate est une solution sans précipités substantiels lorsque soumis à la séparation pour éliminer l'anhydrase carbonique ou l'analogue de celle-ci.

10. Le procédé de la revendication 9, dans lequel la solution sans précipités substantiels est obtenue en fournissant la solution à une température suffisante pour solubiliser le bicarbonate.

11. Le procédé de la revendication 10, dans lequel la température suffisante est atteinte en chauffant le flux chargé de bicarbonate.

12. Le procédé de la revendication 1, dans lequel le flux chargé de bicarbonate est une solution sans précipités substantiels, obtenue en fournissant la solution à une température suffisante pour solubiliser le bicarbonate lorsque soumis à la séparation pour éliminer l'anhydrase carbonique ou l'analogue de celle-ci et dans lequel, après la séparation pour éliminer l'anhydrase carbonique ou l'analogue de celle-ci, le bicarbonate précipité est obtenu en refroidissant la solution.

13. Le procédé de la revendication 1, comprenant en outre une précipitation du bicarbonate pour éviter un piégeage substantiel et/ou une adsorption substantielle de l'anhydrase carbonique ou de l'analogue de celle-ci dans le bicarbonate précipité.

14. Le procédé de l'une quelconque des revendications 1 à 13, dans lequel le carbonate est le carbonate de sodium, potassium, césium ou ammonium, et le composé bicarbonate est le bicarbonate de sodium, potassium, césium ou ammonium.

15. Le procédé de l'une quelconque des revendications 1 à 13, dans lequel le carbonate est le carbonate de sodium et le composé bicarbonate est le bicarbonate de sodium.
